# EUROPEAN PATENT APPLICATION

(11) **EP 1 935 417 A1**
(43) Date of publication of application: **25.06.2008**
(21) Application number: 06798483.1
(22) Date of filing: 29.09.2006
(51) Int. Cl.: A61K 31/198, A61K 31/7004, A61P 25/00, A61P 25/02, A61P 43/00

(54) **COMPOSITION FOR USE IN PREVENTION OF HYPOGLYCEMIC CONDITION**

(30) Priority: 12.10.2005 JP 2005298089
(71) Applicant: OTSUKA PHARMACEUTICAL FACTORY, INC., Naruto-shi, Tokushima 772-8601 (JP)
(72) Inventor: DOI, Masako, Naruto-shi, Tokushima 7720003 (JP); YAMAOKA, Ippei, Tokushima-shi, Tokushima 7700861 (JP)
(74) Representative: Findeisen, Marco
(86) International application number: PCT/JP2006/319574
(87) International publication number: WO 2007/043363

(57) **Abstract**

Disclosed is a composition for use in the prevention of a hypoglycemic condition, which comprises at least one compound selected from a branched amino acid, a pharmaceutically acceptable salt thereof and a derivative thereof as an active ingredient. The composition can promote the uptake of carbohydrate into brain cells to thereby prevent a hypoglycemic condition.

## Description

### TECHNICAL FIELD

The present invention relates to a composition for preventing a hypoglycemic condition or a promoter for carbohydrate uptake into brain cells, the composition or the promoter comprising a branched amino acid or a pharmaceutically acceptable salt thereof, or a derivative thereof as an active ingredient.

### BACKGROUND ART

A hypoglycemic condition refers to a condition where a reduction in glucose level in blood is accompanied by a reduction in glucose level in the brain thereby causing lassitude, general discomfort, dismay, malaise, jitteriness, trembling, headache, weakness, cold sweat and palpitation, additionally causing impaired consciousness and coma, which may also lead to death in a serious case.

The causes of the hypoglycemic condition mainly include the following cases:
(1) the case where a hypoglycemic agent (insulin etc.) is administered in excess to a patient with diabetes;
(2) the case where a certain kind of pharmaceutical produces a side effect;
(3) the case where eating habit of ingesting a large amount of carbohydrate is continued for a long time;
(4) the case where excessive alcohol is ingested; and
(5) the case where extreme exercise is continued for a long time in a state of dietary insufficiency.

Particularly the case (1) or (2) is extremely dangerous because blood sugar is compulsorily reduced by a drug, resulting in a severe condition with high possibility.

Conventionally, in order to simply prevent or treat a hypoglycemic condition, glucose, sugar or carbohydrates has been generally taken. However, ingestion of carbohydrates may exert a harmful influence on the treatment of a patient with diabetes and cause even a healthy individual to take excessive calorie.

An agent for relieving muscular pain and muscle strain and stiffness by oral ingestion of a branched amino acid (see JP-A 2000-26289) and an agent for maintaining instantaneous or sustaining muscular power during exercise by oral ingestion a branched amino acid (see JP-A 2000-26290) are known as a drug containing a branched amino acid that is the active ingredient of the present invention. By focusing on the fact that branched amino acids such as leucine, isoleucine and valine have organ-specificity that can be utilized mainly in muscles and in tissues such as kidney tissue other than liver tissue, it is found that the branched amino acids are useful in ameliorating muscular pain and muscle stiffness or in maintaining instantaneous or sustaining muscular power during exercise. In addition, an agent for relieving fatigue in CNS comprising a branched amino acid (see WO2002/034257) or the like is known. This elucidates the mechanism of fatigue in CNS and simultaneously demonstrates that 2-aminobicyclo[2,2,1]heptane-2-carboxylic acid that is a specific inhibitor of L-system transporter in the blood-brain barrier, can suppress the fatigues in CNS, particularly almost completely when used in combination with a branched amino acid such as leucine, isoleucine or valine. A composition for improving brain cell metabolism (see JP-A 2-172915) and an anti-dementia drug (see JP-A 3-275631), both containing a branched amino acid, are known. However, these documents do not describe that branched amino acids promote incorporation of glucose into cells such as brain cells in hypoglycemic conditions.

Known pharmaceutical preparations containing branched amino acids include a pharmaceutical jelly containing only a branched amino acid as an active ingredient, which is administered in a smaller amount and is excellent in flavor and in swallowing (see JP-A 2003-221330) ; a pharmaceutical dry syrup containing a branched amino acid, a suspending agent and a surfactant, which has improved flavor and feel upon drinking and, upon being suspended, keep excellent suspensibility (see JP-A 2003-221329) ; and a chewable tablet containing, as an active ingredient, a branched amino acid excellent in storage stability without coloration during storage (see JP-A 2003-221327). However, none of these documents describes promotion of carbohydrate incorporation into brain cells or prevention of hypoglycemic conditions.

### DISCLOSURE OF THE INVENTION

### PROBLEMS TO BE SOLVED BY THE INVENTION

The object of the present invention is to provide a composition capable of suppressing a hypoglycemic condition by promoting incorporation of a carbohydrate into brain cells.

### MEANS FOR SOLVING THE PROBLEMS

The present inventors made extensive examination to solve the problem described above. As a result, they found that a branched amino acid or a pharmacologically acceptable salt thereof, or a derivative thereof has a promoting activity on incorporating a carbohydrate into brain cells, and thus can suppress a hypoglycemic condition, and the present invention was thereby completed.

That is, the present invention relates to:
(1) a composition for preventing a hypoglycemic condition, comprising at least one compound as an active ingredient selected from a branched amino acid, a pharmaceutically acceptable salt thereof and a derivative thereof;
(2) the composition according to the above-mentioned (1), wherein the branched amino acid is at least one compound selected from L-valine, L-leucine and L-isoleucine;
(3) the composition according to the above-mentioned (2), comprising at least L-isoleucine;
(4) the composition according to the above-mentioned (3), wherein the content ratio of L-isoleucine, L-leucine and L-valine in terms of molar ratio is 1 : (0 to 3) : (0 to 2);
(5) the composition according to any one of the above-mentioned (1) to (4), further comprising a carbohydrate;
(6) the composition according to the above-mentioned (5), wherein the carbohydrate is glucose;
(7) the composition according to any one of the above-mentioned (1) to (6), wherein the hypoglycemic condition is a sympathetic stimulation condition and/or a cerebral symptom;
(8) a promoter for incorporating a carbohydrate into brain cells, comprising at least one compound selected from a branched amino acid, a pharmaceutically acceptable salt thereof and a derivative thereof as an active ingredient;
(9) the promoter for incorporating a carbohydrate into brain cells according to the above-mentioned (8), wherein the branched amino acid is at least one compound selected from L-valine, L-leucine and L-isoleucine;
(10) the promoter for incorporating a carbohydrate into brain cells according to the above-mentioned (9), comprising at least L-isoleucine;
(11) the promoter for incorporating a carbohydrate into brain cells according to the above-mentioned (8), wherein the content ratio of L-isoleucine, L-leucine and L-valine in terms of molar ratio is 1 : (0 to 3) : (0 to 2);
(12) the promoter for incorporating a carbohydrate into brain cells according to any one of the above-mentioned (8) to (11), further comprising a carbohydrate;
(13) the promoter for incorporating a carbohydrate into brain cells according to the above-mentioned (12), wherein the carbohydrate is glucose;
(14) the composition according to any one of the above-mentioned (1) to (7), which is in the form of an injection or granules;
(15) the promoter for incorporating a carbohydrate into brain cells according to any one of the above-mentioned (8) to (13), which is in the form of an injection or granules;
(16) a food comprising at least one compound selected from a branched amino acid, a pharmaceutically acceptable salt thereof and a derivative thereof, suppressing a hypoglycemic condition or promoting the incorporation of a carbohydrate into brain cells;
(17) the food according to the above-mentioned (16), wherein the food is a jelly;
(18) a method for inhibiting a hypoglycemic condition, comprising administering at least one compound selected from a branched amino acid, a pharmaceutically acceptable salt thereof and a derivative thereof to a mammal;
(19) a method for promoting the incorporation of a carbohydrate into brain cells, comprising administering at least one compound selected from a branched amino acid, a pharmaceutically acceptable salt thereof and a derivative thereof to a mammal;
(20) use of at least one compound selected from a branched amino acid, a pharmaceutically acceptable salt thereof and a derivative thereof, for producing a composition to prevent a hypoglycemic condition; and
(21) use of at least one compound selected from a branched amino acid, a pharmaceutically acceptable salt there of and a derivative thereof for producing a promoter to incorporate a carbohydrate into brain cells.

### EFFECT OF THE INVENTION

According to the present invention, the incorporation of carbohydrates into brain cells can be promoted, and thus a reduction in carbohydrate levels in brain cells can be prevented, and as a result, conditions accompanying low blood sugar can be suppressed.

The composition for prevention of a hypoglycemic condition or the promoter for incorporation of carbohydrates into brain cells according to the present invention can effectively be used in patients with diabetes who are prone to fall into hypoglycemia upon insulin injection or antidiabetic medication, for the composition or the promoter hardly changes blood glucose level.

The composition for prevention of a hypoglycemic condition or the promoter for incorporation of carbohydrates into brain cells according to the present invention promotes incorporation of glucose, the energy source for brain cells, and thus can suppress not only the above-mentioned conditions attributable to hypoglycemia but also hypoglycemic-like conditions which is caused, even when blood glucose level is normal, by glucose level reduction.

### BRIEF DESCRIPTION OF DRAWINGS

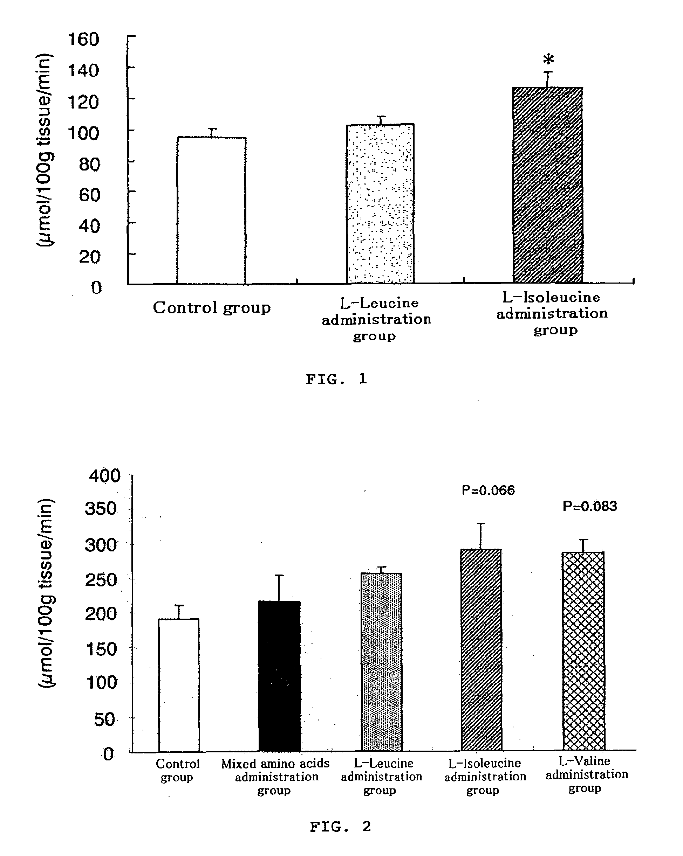

FIG. 1 is a graph showing the amount of a carbohydrate incorporated into rat brain cells 1 hour after oral administration of L-leucine or L-isoleucine with a control group as a comparative example. In the graph, the ordinate shows the amount of 2DG-6P, and * shows a significant difference (p<0.05) relative to the control group. Each column value shows the mean (n = 6) ± standard error.
FIG. 2 is a graph showing the amount of a carbohydrate incorporated into rat brain cells at 24 hours after intravenous administration of mixed amino acids, L-leucine, L-isoleucine or L-valine with a control group as a comparative example. In the graph, the ordinate shows the amount of 2DG-6P. Each column value shows the mean (n = 4) ± standard error.

### BEST MODE FOR CARRYING OUT THE INVENTION

Hereinafter, the composition for prevention of a hypoglycemic condition or the promoter for incorporation of a carbohydrate into brain cells which comprises at least one compound selected from a branched amino acid, a pharmaceutically acceptable salt of the branched amino acid, and a derivative of the branched amino acid or the pharmaceutically acceptable salt thereof as an active ingredient according to the present invention will be described in detail.

The branched amino acids used in the present invention are not particularly limited so long as they meet the standards of the Japanese Pharmacopoeia, and examples of such branched amino acids include any branched amino acids such as L-amino acids, D-amino acids, α-amino acids, β-amino acids, γ-amino acids, natural amino acids, synthetic amino acids and the like, preferably natural L-amino acids or α-amino acids. Particularly preferable examples of the branched amino acids used in the present invention include L-valine, L-leucine and L-isoleucine. In addition, the above-mentioned branched amino acids may be prepared by hydrolyzing plant-derived or animal-derived proteins with a protease, or by microbial fermentation methods, or may be synthetic amino acids which are prepared by introducing amino groups into organic acids, etc.

Pharmaceutically acceptable salts of the branched amino acids in the present invention are salts of acids or bases and are not particularly limited, and examples thereof include alkali metal salts such as sodium salts and potassium salts, alkaline earth metal salts such as calcium salts, inorganic acid salts such as hydrochlorides , and organic acid salts such as acetates, and among them hydrochlorides are preferable. Specific examples include L-valine hydrochloride, L-leucine hydrochloride and L-isoleucine hydrochloride.

Derivatives of the branched amino acids or the pharmaceutically acceptable salts thereof used in the present invention are not particularly limited, and examples include esters and peptides. The esters are not particularly limited as long as they are lower alkyl esters such as methyl ester, ethyl ester, propyl ester or isopropyl ester. The lower alkyl in this case is preferably an alkyl group containing 1 to 6 carbon atom(s). Preferable examples of the esters include L-valine ethyl ester, L-leucine ethyl ester and L-isoleucine ethyl ester. The peptides are not particularly limited insofar as they are oligopeptides such as dipeptides and tripeptides, and preferable examples of the peptides include L-isoleucyl-L-leucine, L-alanyl-L-leucine, L-leucyl-L-alanine, glycyl-L-leucine and L-alanyl-L-glutamine. Accordingly, those amino acids other than the branched amino acid that constitute the oligopeptides may be any amino acids including L-amino acids, D-amino acids, α-amino acids, β-amino acids, γ-amino acids, natural amino acids, synthetic amino acids and the like.

These branched amino acids or pharmaceutically acceptable salts thereof or derivatives thereof can be used alone or as a mixture of two or more thereof, but at least L-isoleucine or a salt thereof or a derivative thereof is preferably contained, and at least L-isoleucine is particularly preferably contained.

The branched amino acid or pharmaceutically acceptable salts thereof or derivatives thereof in the composition of the present invention can be used alone or as a mixture of two or more thereof. When two or more of these are used, their mixing ratio is not particularly limited. Specifically when the branched amino acid or pharmaceutically acceptable salts thereof or derivatives thereof , for example L-isoleucine, L-leucine or L-valine is used, these compounds may be used alone or as a mixture of two or more thereof, and the mixing ratio of the compounds, in terms of molar ratio, is L-isoleucine : L-leucine : L-valine = about 1 : (0 to 3) : (0 to 2), more preferably about 1 : (0 to 2.5) : (0 to 1.5). For example, when salts of L-isoleucine, L-leucine or L-valine or derivatives thereof are used, they are used preferably in the above molar ratio in terms of L-isoleucine, L-leucine and L-valine, respectively.

The hypoglycemic condition in the present invention refers to a condition caused by a reduction in glucose level in blood. Glucose is the most important energy source for every cell, and particularly the brain depends exclusively on glucose for energy. Glucose is essential also for muscles and other tissues to function. Therefore, when glucose levels in blood are reduced, there occur conditions undesirable for the functions of the brain, muscles and other tissues. Such conditions include, but are not limited to, a sympathoexcitatory condition and a cerebral symptom. The sympathoexcitatory condition includes, for example, dismay, hunger, stagger, dizziness, anemia, weakness, lassitude, slight yawn, aggravation, trembling, palpitation, facial pallor, tachycardia, sweating and tremor, and the cerebral symptom includes, for example, headache, visual disorder, double vision, mist, decline in concentration power and in calculating ability, amnesia, lethargy, impaired consciousness,convulsion, nerve abnormality, physical abnormal behavior, and coma.

As used herein, hypoglycemia includes a state of a blood glucose level of not higher than about 60 mg/dL, but is not limited to this blood glucose level. For example, when a person having high blood glucose due to diabetes or the like undergoes a reduction in blood glucose level upon insulin injection or the administration of an antidiabetic agent, or when a healthy individual undergoes rapid reduction in blood glucose level due to hunger or fast and furious exercise, similar conditions to hypoglycemia can appear even at about 100 mg/dL, and such conditions similar to hypoglycemia are also included in the present invention.

The composition for prevention of a hypoglycemic condition in the present invention refers to a composition for suppressing or ameliorating a condition caused by reduced glucose levels in blood.

The composition for prevention of a hypoglycemic condition according to the present invention can be used not only in a hypoglycemic condition but also prophylactically even in a non-hypoglycemic state.

The promoter for incorporation of carbohydrates into brain cells according to the present invention promotes incorporation of carbohydrates, particularly glucose as a sole energy source in the brain, into brain cells, thus enabling suppression of a state where the incorporation of glucose into the brain is suppressed, for example, the above-mentioned hypoglycemic condition or a condition which, for some reasons except hypoglycemia, causes a condition similar to hypoglycemia.

Also, the promoter for incorporation of carbohydrates into brain cells is expected to promote incorporation of carbohydrates into cells that are not brain cells, for example muscular cells, nerve cells, and other cells, and can thus be expected to activate the muscular cells, nerve cells and other cells.

Preferably, the composition for prevention of a hypoglycemic condition or the promoter for incorporation of carbohydrates into brain cells according to the present invention further contains a carbohydrate. The carbohydrate includes, but is not limited to, monosaccharides such as ribose, deoxyribose, glucose, fructose and galactose; disaccharides such as maltose, sucrose and lactose; and polysaccharides such as amylose, amylopectin and glycogen. Among the carbohydrates, monosaccharides are more preferable, and glucose is particularly preferable because it can serve as an immediate energy source utilized within a living organism.

In this case, the content ratio of carbohydrates is not particularly limited, but is preferably for example about 0.1 to 50 moles in terms of glucose per mole of branched amino acid.

The composition for prevention of a hypoglycemic condition or the promoter for incorporation of carbohydrates into brain cells according to the present invention can be provided in the form of a drug containing pharmaceutically acceptable additives or in the form of a food containing additives approved under the food sanitation law.

The composition for prevention of a hypoglycemic condition or the promoter for incorporation of carbohydrates into brain cells according to the present invention, when formed in the form of a pharmaceutical preparation, can be used for example as a solid preparation for oral administration, a liquid medicine for oral administration, or an injection for parenteral administration (subcutaneous, intravenous, intramuscular, intraperitoneal injection etc.). The solid preparation for oral administration includes, for example, tablets, pills, capsules, powder and granules.

The additives that can be used in the solid preparation for oral administration include, for example, an excipient, a binder, a disintegrating agent, a lubricant, a stabilizer and a wetting agent. The excipient includes, but is not limited to, sucrose, lactose, glucose, starch and mannitol. The binder includes, but is not limited to, gum arabic , carmellose, gelatin, crystalline cellulose, hydroxypropyl cellulose, methyl cellulose and popidone. The disintegrating agent includes, but is not limited to, carmellose, starch, crystalline cellulose and low-substituted hydroxypropyl cellulose. The lubricant includes, but is not limited to, talc, magnesium stearate, calcium stearate and silica. The stabilizer and the wetting agent include, but are not limited to, citric anhydride, sodium laurate and glycerol. These additives can be used alone or as a mixture of two or more thereof. The solid preparation for oral administration can be prepared for example by mixing a branched amino acid, a salt thereof or a derivative thereof with additives and forming the mixture into a pharmaceutical preparation according to methods described in, for example, general rules in the Japanese Pharmacopoeia, 14^{th} revised edition. Specifically, the granules can be prepared for example by adding the above-described excipient, binder, disintegrating agent etc. to , a salt thereof or a derivative thereof, kneading the mixture uniformly, and then forming the mixture into granules preferably through compression granulation, tumbling granulation, spray drying granulation, extrusion granulation, milling granulation, fluidization granulation or agitation granulation. The tablets can be produced for example by adding the above-described excipient, binder, disintegrating agent etc. to a branched amino acid, a salt thereof or a derivative thereof and kneading the mixture uniformly followed by direct compression molding, or produced by preparation of granulates from a branched amino acid, a salt thereof or a derivative thereof and the excipient, binder, disintegrating agent etc. followed by forming the granules as they are into tablets, or produced by uniformly mixing the active ingredient with the additives and then forming the mixture into tablets by compression molding. The granules or tablets may be coated if necessary with a suitable coating (gelatin, sucrose, gum arabic, carnauba wax etc.) or an enteric coating (for example, cellulose acetate phthalate, a methacrylic acid copolymer, hydroxypropyl cellulose phthalate, carboxymethylethyl cellulose, etc.). The capsules can be produced for example by adding the above-described excipient, binder, disintegrating agent etc. to a branched amino acid, a salt thereof or a derivative thereof and kneading the mixture uniformly, then optionally formed into granules, or, coating formed granules with a coating followed by being charged into capsules.

The content ratio of a branched amino acid, a salt thereof or a derivative thereof in the solid preparation for oral administration is not particularly limited, but preferably the total amount of branched amino acids is about 1 to 90% by mass based on the whole of the solid preparation.

The liquid medicine for oral administration includes, for example, a drench, a suspension, an emulsion, a syrup and an elixir. Additives usable in such liquid medicine include, for example, purified water, ethanol, and a solvent such as a mixture thereof. The liquid medicine for oral administration may contain a suspending agent (for example, gum arabic, agar, carmellose, hydroxypropyl cellulose, etc.), an emulsifying agent (for example, polysorbate 80, gum arabic, etc.), a flavoring substance (for example, simple syrup, honey, sucrose, tartaric acid etc.), an aromatic substance (for example, methyl salicylate, fennel oil, orange oil, menthol etc.), a preservative (for example, aromatic acid, sodium benzoate, etc.) and a buffering agent (for example, citric acid, hydrogen carbonate, etc.). These additives can be used alone or as a mixture of two or more thereof.

Additives that can be used in an injection for parenteral administration include, for example, a solvent, a stabilizer, a solubilizing agent, a suspending agent, a surfactant, an emulsifying agent, soothing agent, a buffering agent and a preservative. The solvent includes, but is not limited to, distilled water for injection, physiological saline, vegetable oil such as sesame oil, ethyl alcohol, isopropyl alcohol, propylene glycol, 1,3-butylene glycol and polyethylene glycol. The stabilizer and the solubilizing agent include, but are not limited to, glutamic acid, aspartic acid and polysorbate 80. The suspending agent includes, but is not limited to, cellulose derivatives such as carboxymethyl cellulose sodium and methyl cellulose, and natural gum such as tragacanth and gum arabic. The surfactant includes, but is not limited to, sorbitan fatty acid ester, polyoxyethylene sorbitan fatty acid ester, polyoxyethylene fatty acid ester, polyoxyethylene ether of hydrogenated castor oil, and lecithin. The emulsifying agent includes, but is not limited to, polyoxyl stearate, lauromacrogol, polysorbate 80 and gum arabic. The soothing agent includes, but is not limited to, ethyl aminobenzoate, inositol, meprilcaine hydrochloride, lidocaine hydrochloride, chlorobutanol, propylene glycol and benzyl alcohol. The buffering agent includes, but is not limited to, citric acid or a salt thereof, glucose, phosphoric acid or a salt thereof, and acetic acid or a salt thereof. The preservative includes, but is not limited to, p-hydroxybenzoate ester, benzalkonium chloride, and sorbitan acid salt. These additives can be used alone or as a mixture of two or more thereof.

The injection can be produced by dissolving a branched amino acid, a salt thereof or a derivative thereof and additives suitably in a usual manner, for example by aseptic manipulation. The produced injection is charged into an ampoule, a vial container, or a glass or polyethylene infusion container (including a bag) and then sterilized. The polyethylene infusion container (including a bag) may be packaged for example in a gas-barrier packing material together with a deoxygenating agent. The injection may be produced in a form of a sterile solid preparation, for example a lyophilized product, which can be used by being dissolved in sterilized or sterile distilled water for injection or in another solvent just before use.

The content ratio of a branched amino acid, a salt thereof or a derivative thereof is not particularly limited, but in the case of an infusion preparation, the total amount of branched amino acids is preferably about 0.1 to 10 w/v% based on the whole of the injection.

The composition for prevention of a hypoglycemic condition or the promoter for incorporation of carbohydrates into brain cells according to the present invention may further contain nutrients such as vitamins (for example, vitamin A, vitamin B₁, B₂, B₆, B₁₂, vitamin C, vitamin D, vitamin E, niacin, pantothenic acid, folic acid, biotin, vitamin F , vitamin P , vitamin Q, vitamin U, choline, inositol, p-aminobenzoic acid, etc.) and amino acids other than branched amino acids (for example, lysine, phenylalanine, methionine, threonine, valine, histidine, tryptophan, alanine, proline, arginine, glutamic acid, serine etc.).

The dosage of the medicine containing the composition for prevention of a hypoglycemic condition or the promoter for incorporation of carbohydrates into brain cells according to the present invention is not particularly limited and may be arbitrarily determined depending on the formulation, administration route, the age and weight of the patient, and the severity of the disease. General dosage per kg of an adult is within the range of about 1 to 1000 mg, preferably about 1 to 500 mg, and may be suitably increased or decreased as desired. The medicine may also be administered in several doses per day.

When the composition for prevention of a hypoglycemic condition or the promoter for incorporation of carbohydrates into brain cells according to the present invention is produced in the form of a food, at least one of a branched amino acid, a salt thereof and a derivative thereof , preferablyL-isoleucine, is mixed with additives approved under the food sanitation law and with other various components used for food to form a food or drink product. The form of the food to be produced is not particularly limited, and the food may be in every possible form such as tablets, capsules, powder, granules, a liquid medicine for oral administration, a solid food, a semi-liquid food in the form of cream or jam, a gelatinous food and a drink. Specific examples of the food include, for example, soft drinks, drinks such as a juice or lactic acid bacteria beverage, jelly, candies, biscuits and cookies. The method for producing the food is not particularly limited, and any means known in the art can be used.

The tablets, capsules, powder, granules or a liquid preparation for oral administration can be produced in the same manner as in the production of the above-described medicine except that additives approved under the food sanitation law (for example, hydroxypropylmethyl cellulose, crystalline cellulose, tartaric acid, mannitol, saccharine sodium, stevia, dimethylpolysiloxane, p-hydroxybenzoate ester etc.) are used in place of the additives used in the tablets, capsules, powder, granules or a liquid medicine for oral administration as the medicine described above.

The drink to be produced may contain additives , for example, flavoring substances and synthetic sweeteners such as a flavor, a coloring matter, natural juice, flesh, cheese and chocolate, and a synthetic sweetener as needed. The additives may be used alone or as a mixture of two or more thereof.

The semi-liquid food in the form of cream or jam or the gelatinous food such as jelly, to be produced, may preferably contain one or more gelling agents selected from agar, gelatin, carrageenan, gellan gum, xanthan gum, locust bean gum, pectin, sodium alginate, potassium alginate and other ordinarily used polysaccharide thickeners, in addition to the above-mentioned components in drink. The blending amount of the gelling agent is about 2 parts or less by mass, preferably about 0.2 to 2 parts by mass per 100 parts by mass of jelly.

The content ratio of a branched amino acid, a salt thereof or a derivative thereof in the food is not particularly limited, but preferably the total amount of branched amino acids is preferably about 1 to 60% by mass.

The food produced in this manner can be used as a functional food for suppressing a hypoglycemic condition or for promoting incorporation of carbohydrates into brain cells. An indication of its effect of suppressing a hypoglycemic condition or promoting incorporation of carbohydrates into brain cells is preferably provided on the packaging of such food.

The food is ingested preferably in a daily amount of about 0.1 to 20 g in terms of branched amino acids, salts thereof or derivatives thereof, per adult (about 60 kg).

### EXAMPLES

Hereinafter, the present invention is described in more detail by reference to Pharmaceutical Examples and Test Examples, but the present invention is not limited to these examples.

### <Production Example 1>

300 g of L-isoleucine was dissolved in distilled water for injection, then adjusted to pH 6.50 with sodium hydroxide and adjusted to a total volume of 10 L with distilled water for injection. The resulting solution was filtered through a membrane filter, introduced in a volume of 500 mL/bag into polyethylene infusion bags, sealed and sterilized at 105°C for 40 minutes by a process of steaming under pressure. The bag, together with a deoxygenating agent (trade name: Ageless, manufactured by Mitsubishi Gas Chemical Co. , Inc.), was packaged in a gas-barrier packaging material made of a polyvinyl alcohol multilayer film, to give an injection.

### <Production Example 2>

150 g of L-isoleucine and 150 g of L-valine were dissolved in distilled water for injection, then adjusted to pH 6.50 with sodium hydroxide and adjusted to a total volume of 10 L with distilled water for injection. The resulting solution was filtered through a membrane filter, introduced in a volume of 500mL/bag into polyethylene infusion bags, sealed and sterilized at 105°C for 40 minutes by a process of steaming under pressure. The bag, together with a deoxygenating agent (trade name: Ageless, manufactured by Mitsubishi Gas Chemical Co. , Inc.), was packaged in a gas-barrier packaging material made of a polyvinyl alcohol multilayer film, to give an injection.

### <Production Example 3>

300 g of L-isoleucine was dissolved in distilled water for injection, then adjusted to pH 6.50 with sodium hydroxide, adjusted to a volume of 10 L with distilled water for injection and filtered through a membrane filter.

Separately, 1000 g of glucose was dissolved in distilled water for injection, then adjusted to a volume of 10 L with distilled water for injection and filtered through a membrane filter.

500 ml each of these solutions were separately introduced into chambers of a polyethylene two-chamber infusion bag, sealed and sterilized at 105°C for 40 minutes by a process of steaming under pressure. The bag, together with a deoxygenating agent (trade name: Ageless, manufactured by Mitsubishi Gas Chemical Co., Inc.), was packaged in a gas-barrier packaging material made of a polyvinyl alcohol multilayer film, to give an injection.

To use this injection, one chamber or two chambers must be pressed, thereby linking the two chambers to each other in order to prepare a mixture to be used as an injection.

### <Production Example 4>

1000 g of L-alanyl-L-leucine, 300 g of L-isoleucine and 240 g of L-valinewere dissolved in distilledwater for injection, then adjusted to pH 6.50 with sodium hydroxide and adjusted to a total volume of 10 L with distilled water for injection. The resulting solution was filtered through a membrane filter, introduced in a volume of 100 mL/bag into polyethylene infusion bags, sealed and sterilized at 105°C for 40 minutes by a process of steaming under pressure. The bag, together with a deoxygenating agent (trade name: Ageless, manufactured by Mitsubishi Gas Chemical Co. , Inc.), was packaged in a gas-barrier packaging material made of a polyvinyl alcohol multilayer film, to give an injection.

### <Production Example 5>

120 g of hydroxypropylmethyl cellulose and 10 g of crystalline cellulose carmellose sodium were added to, and dispersed in, 10 L of purified water. To this dispersion, 100 g of tartaric acid, 1000 g of mannitol, 10 g of saccharine sodium, 5 g of stevia, 40 g of dimethylpolysiloxane, 2 g of propyl p-hydroxybenzoate and 5 g of methyl p-hydroxybenzoate, and then these ingredients were added and dissolved. Subsequently, 950 g of L-isoleucine, 1900 g of L-leucine and 1150 g of L-valine were added thereto, suspended with a homogenizer, adjusted to pH 6.5 with sodium hydroxide and uniformly suspended with a homogenizer to prepare a suspension.

Separately, 40 g of agar powder was placed in 2 L of purified water and dissolved by heating at about 80°C, and 6000 g of the above suspension and 40 g of a pineapple flavor were added to, and mixed with, the resulting solution. The resulting mixture was introduced in an amount of 100 g/container into containers, sealed and chilled to prepare a jelly.

### <Production Example 6>

500 g of L-isoleucine, 1000 g of L-leucine, 600 g of valine, 100 g of citric anhydride and 50 g of hydroxypropyl cellulose were mixed uniformly, and then 300 g of distilled water was added to the mixture, which was then granulated.

The granulated product was dried at 60°C for 2 hours and passed through a 24-mesh sieve to give granules. The resulting granules were introduced in an amount of 4.5 g/bag into aluminum laminate stick bags and sealed to give a final product. <Test Example 1> Promoting activity of orally administered branched amino acid (also referred to hereinafter as BCAA) in incorporation of carbohydrate into the brain

### (1) Preparation of test solutions

Leucine group: An L-leucine suspension prepared by adding 3 g L-leucine to 100 mL physiological saline and suspending it with a homogenizer was used as a test solution of leucine group. Isoleucine group: An L-isoleucine suspension prepared by adding 3 g L-isoleucine to 100 mL physiological saline and suspending it with a homogenizer was used as a test solution of isoleucine group.

### (2) Test method

Male 6-week-old Wistar rats (Charles River Japan, Inc.) were kept for 14 days and given free access to food (purified feed: AIN-93G manufactured by Nippon Nosan Kogyo Co., Ltd.) and water. At the age of 8-week-old, the rats were grouped depending on the body weight into 3 groups (control group, leucine group and isoleucine group). After the grouping, under ether anesthesia, the cervical region of each rat was opened, and a catheter was inserted into the jugular vein. The other end of the catheter was passed subcutaneously, exposed on the back and connected via a harness to, and fixed to, a swivel. Then each rat was housed in a separate metabolism cage. Physiological saline was administered via the catheter continuously at a rate of 1 mL/hr/body to the rat. The next day, the control group was orally administered with physiological saline at a dose of 15 mL/kg BW (body weight), while the leucine group and the isoleucine group were orally administered with their corresponding test solutions, that is, the L-leucine suspension and L-isoleucine suspension, at a dose of 0.45 g/15 mL/kg BW. 20 minutes after administration of the test solution, 30 µCi/kg 2-[1,2-³H]-deoxyglucose (2DG) was administered intravenously via the catheter over about 3 seconds. 2, 10, 20, 30 and 40 minutes after intravenous administration, blood was collected in a volume of 300 µL via the catheter and then treated with heparin followed by separation of blood plasma. The separated plasma was subjected to measurement of 2DG level and blood glucose level. 20 µL of the plasma was placed in a vial for liquid scintillation counter measurement, and then 5 mL scintillator was added to and mixed with the plasma, which was then measured for its tritium level (count) with a liquid scintillation counter to determine the amount of 2DG. The blood glucose level was measured by a glucose oxidase dye method.

One hour after administration of the test solution, the rats were administered intravenously with pentobarbital sodium in a dose of 50 mg/kg over about 3 seconds, and under anesthesia, blood was removed via an abdominal aorta by blood collection with heparin. After blood removal, the brain tissue was removed, mixed with 1N NaOH that was 4 parts of the brain tissue by weight, and then heated in a water bath at 60°C for 1 hour to lyse the brain tissue. To the brain tissue lysate solution, 1N HCl in the same amount as that of 1N NaOH was added, thereby neutralizing the solution to give a neutralized homogenate of the brain tissue. Amount of 2DG in the neutralized homogenate: To 400 µL of 0.15 N ZnSO₄,400 µL of the neutralized homogenate was added, and then 400 µL of 0.15 N Ba (OH)₂ was added and mixed. After centrifugation (6000 rpm, 20 minutes), a supernatant was separated. 900 µL of the separated supernatant was placed in a vial for liquid scintillation counter measurement, and then 5 mL scintillator was added to and mixed with the supernatant, which was then measured for its tritium level (count) with a liquid scintillation counter to determine the amount of 2DG.
Amount of 2DG and 2DG-6P(2-[1,2-³H]-deoxyglucose-6-phosphate), in the neutralized homogenate: To 400 µL of the neutralized homogenate, 800 µL of 6 wt% PCA (perchloric acid) was added and mixed. After centrifugation (6000 rpm, 20 minutes), a supernatant was separated. 900 µL of the separated supernatant was placed in a vial for liquid scintillation counter measurement, and then 10 mL scintillator was added to and mixed with the supernatant, which was then measured for its tritium level (count) with a liquid scintillation counter to determine the amount of 2DG and 2DG-6P.
Amount of 2DG-6P in the neutralized homogenate: In order to determine the amount of 2DG-6P, the amount of 2DG was subtracted from the amount of 2DG and 2DG-6P. The determined amount of 2DG-6P was corrected with blood 2DG level and blood glucose level, to determine the amount of the carbohydrate incorporated into the brain.

In statistical processing, after analysis of variance, Dunnett's multiple comparison with the control was carried out, and p < 0.05 was regarded as significantly different.

### (3) Test Results

The results are shown in FIG. 1.

The results revealed that the amount of the carbohydrate incorporated into brain cells 1 hour after the oral administration of the test solution, L-leucine suspension or L-isoleucine suspension, was higher than that of the control group. Particularly, it was found that the incorporation of the carbohydrate into brain cells was significantly promoted in the isoleucine group as compared with the control group. <Test Example 2> Promoting activity of intravenously administered BCAA in incorporation of carbohydrate into the brain (1) Preparation of test solutions
Control group: A solution prepared by dissolving glucose at a concentration of 5% by mass in purified water was used as the test solution for the control group.
Mixed amino acid group: A solution prepared by dissolving Amiparen (registered trademark, manufactured by Otsuka Pharmaceutical Co., Ltd.) at a concentration of 1.5% by mass in purified water containing 5% by mass glucose was used as the test solution for the mixed amino acid group.
Leucine group: A solution prepared by dissolving L-leucine at a concentration of 1.5% by mass in purified water containing 5% by mass glucose was used as the test solution for the leucine group.
Isoleucine group: A solution prepared by dissolving L-isoleucine at a concentration of 1.5% by mass in purified water containing 5% by mass glucose was used as the test solution for the isoleucine group.
Valine group: A solution prepared by dissolving L-valine at a concentration of 1.5% by mass in purified water containing 5% by mass glucose was used as the test solution for the valine group.

### (2) Test method

Male 7-week-old SD rats (Nippon Clare) were kept for 14 days and given free access to food (purified feed: AIN-93G manufactured by Nippon Nosan Kogyo Co., Ltd.) and water. At the age of 8-week-old, the rats were grouped depending on the body weight into 5 groups (control group, mixed amino acid group, leucine group, isoleucine group and valine group). After the grouping, under ether anesthesia, the cervical region of each rat was opened, and a catheter was inserted into the jugular vein. The other end of the catheter was passed subcutaneously, exposed on the back and connected via a harness to, and fixed to, a swivel. Then each rat was housed in a separate metabolism cage. The control group was administered via the catheter with 5% by mass glucose solution, while the mixed amino group, the leucine group, the isoleucine group and the valine group were administered with their corresponding test solutions at a rate of 10 mL/kg/hr. 23 hours after administration of the test solution, 30 µCi/kg 2-[1,2-³H]-deoxyglucose (2DG) was administered intravenously via the catheter over about 3 seconds. 2, 10, 20, 30, 40 and 60 minutes after intravenous administration, blood was collected in a volume of 300 µL via the catheter and then treated with heparin followed by separating blood plasma. The separated plasma was measured for its 2DG level and blood glucose level in the same manner as in the test method in Test Example 1.

One hour after administration of 2-[1,2-³H]-deoxyglucose, the rats were administered intravenously with pentobarbital sodium in a dose of 50 mg/kg in about 3 seconds, and under anesthesia, blood was removed via an abdominal aorta by blood collection with heparin. After blood removal, the brain tissue was removed, mixed with 1N NaOH that was 4 parts of the brain tissue by weight, and then heated in a water bath at 60°C for 1 hour to lyse the brain tissue. To the brain tissue lysate solution, 1N HCl in the same amount as that of 1N NaOH was added, thereby neutralizing the solution to give a neutralized homogenate of the brain tissue. The amount of 2DG-6P in the neutralized homogenate was measured in the same manner as in the test method in Test Example 1, to determine the amount of the carbohydrate incorporated into the brain cells.

In statistical processing, after analysis of variance, Dunnett's multiple comparison with the control was carried out to test significant difference.

### (3) Test Results

The results are shown in FIG. 2. The results revealed that the amount of the carbohydrate incorporated into brain cells at 24 hours after the administration of L-leucine, L-isoleucine or L-valine was higher than that of the control group and of the mixed amino acid group. Particularly, the tendency to promote incorporation of the carbohydrate into brain cells was recognized in the isoleucine group and the valine group as compared with the control group.

### INDUSTRIAL APPLICABILITY

According to the present invention, the incorporation of carbohydrates into brain cells can be promoted, thus preventing a reduction of carbohydrate levels in brain cells, resulting in suppressing a hypoglycemic condition, and thus the present invention is useful for a drug or a functional food. The composition for prevention of a hypoglycemic condition and the promoter for incorporation of carbohydrates into brain cells according to the present invention can increase the amount of carbohydrates in brain cells without substantially changing blood glucose levels, and can be used effectively patients with diabetes who are prone to fall into hypoglycemia upon insulin injection or antidiabetic medication.

## Claims

1. A composition for preventing a hypoglycemic condition, comprising at least one compound as an active ingredient selected from a branched amino acid, a pharmaceutically acceptable salt thereof and a derivative thereof.

2. The composition according to claim 1, wherein the branched amino acid is at least one compound selected from L-valine, L-leucine and L-isoleucine.

3. The composition according to claim 2, comprising at least L-isoleucine.

4. The composition according to claim 3, wherein the content ratio of L-isoleucine, L-leucine and L-valine in terms of molar ratio is 1 : (0 to 3) : (0 to 2).

5. The composition according to any one of claims 1 to 4, further comprising a carbohydrate.

6. The composition according to claim 5, wherein the carbohydrate is glucose.

7. The composition according to any one of claims 1 to 6, wherein the hypoglycemic condition is a sympathetic stimulation condition and/or a cerebral symptom.

8. A promoter for incorporating a carbohydrate into brain cells, comprising at least one compound selected from a branched amino acid, a pharmaceutically acceptable salt thereof and a derivative thereof as an active ingredient.

9. The promoter for incorporating a carbohydrate into brain cells according to claim 8, wherein the branched amino acid is at least one compound selected from L-valine, L-leucine and L-isoleucine.

10. The promoter for incorporating a carbohydrate into brain cells according to claim 9, comprising at least L-isoleucine.

11. The promoter for incorporating a carbohydrate into brain cells according to claim 10, wherein the content ratio of L-isoleucine, L-leucine and L-valine in terms of molar ratio is 1 : (0 to 3) : (0 to 2).

12. The promoter for incorporating a carbohydrate into brain cells according to any one of claims 8 to 11, further comprising a carbohydrate.

13. The promoter for incorporating a carbohydrate into brain cells according to claim 12, wherein the carbohydrate is glucose.

14. The composition according to any one of claims 1 to 7, which is in the form of an injection or granules.

15. The promoter for incorporating a carbohydrate into brain cells according to any one of claims 8 to 13, which is in the form of an injection or granules.

16. A food comprising at least one compound selected from a branched amino acid, a pharmaceutically acceptable salt thereof and a derivative thereof, suppressing a hypoglycemic condition or promoting the incorporation of a carbohydrate into brain cells.

17. The food according to claim 16, wherein the food is a jelly.

18. A method for inhibiting a hypoglycemic condition, comprising administering at least one compound selected from a branched amino acid, a pharmaceutically acceptable salt thereof and a derivative thereof to a mammal.

19. A method for promoting the incorporation of a carbohydrate into brain cells, comprising administering at least one compound selected from a branched amino acid, a pharmaceutically acceptable salt thereof and a derivative thereof to a mammal.

20. Use of at least one compound selected from a branched amino acid, a pharmaceutically acceptable salt thereof and a derivative thereof, for producing a composition to prevent a hypoglycemic condition.

21. Use of at least one compound selected from a branched amino acid, a pharmaceutically acceptable salt thereof and a derivative thereof for production of a promoter to incorporate a carbohydrate a carbohydrate into brain cells.
